# EUROPÄISCHE PATENTSCHRIFT

(11) **EP 1 994 173 B1**
(45) Veröffentlichungstag und Bekanntmachung des Hinweises auf die Patenterteilung: **29.08.2012**
(21) Anmeldenummer: 07723318.7
(22) Anmeldetag: 16.03.2007
(51) Int. Cl.: C12Q 1/68

(54) **VERFAHREN FÜR DIE DIAGNOSE UND/ODER DIE PROGNOSESTELLUNG BEI EIERSTOCKKREBS**
METHOD FOR DIAGNOSING AND/OR MAKING PREDICTIONS ABOUT OVARIAN CANCER
PROCÉDÉ POUR FAIRE UN DIAGNOSTIC ET/OU UN PRONOSTIC POUR UN CANCER DES OVAIRES

(30) Priorität: 16.03.2006 AT 4412006
(43) Veröffentlichungstag der Anmeldung: 26.11.2008
(73) Patentinhaber: OncoLab Diagnostics GmbH, 2700 Wiener Neustadt (AT)
(72) Erfinder: KRAINER, Michael, A-9020 Klagenfurt (AT); PILS, Dietmar, A-1180 Wien (AT); ZEILLINGER, Robert, A-2602 Blumau-Neurisshof (AT)
(74) Vertreter: Redl, Gerda
(86) Internationale Anmeldenummer: PCT/EP2007/002338
(87) Internationale Veröffentlichungsnummer: WO 2007/104571

(56) Entgegenhaltungen:
- PILS DIETMAR ET AL: "Five genes from chromosomal band 8p22 are significantly down-regulated in ovarian carcinoma - N33 and EFA6R have a potential impact on overall survival" CANCER, Bd. 104, Nr. 11, Dezember 2005 (2005-12), Seiten 2417-2429, XP002449272 ISSN: 0008-543X
- WEI S H ET AL: "ABERRANT DNA METHYLATION ION OVARIAN CANCER. IS THERE AN EPIGENETIC PREDISPOSITION TO DRUG RESPONSE?" ANNALS OF THE NEW YORK ACADEMY OF SCIENCES, NEW YORK ACADEMY OF SCIENCES, NEW YORK, NY, US, Bd. 983, März 2003 (2003-03), Seiten 243-250, XP001204325 ISSN: 0077-8923
- PLUMB JANE A ET AL: "Reversal of drug resistance in human tumor xenografts by 2'-deoxy-5-azacytidine-induced demethylation of the hMLH1 gene promoter" CANCER RESEARCH, Bd. 60, Nr. 21, 1. November 2000 (2000-11-01), Seiten 6039-6044, XP002449273 ISSN: 0008-5472
- PILS DIETMAR ET AL: "Hypermethylation of N33 (TUSC3) as independent molecular predictor for ovarian cancer." PROCEEDINGS OF THE AMERICAN ASSOCIATION FOR CANCER RESEARCH ANNUAL MEETING, Bd. 47, April 2006 (2006-04), Seite 760, XP001536549 & 97TH ANNUAL MEETING OF THE AMERICAN-ASSOCIATION-FOR-CANCER-RESEARCH (AACR); WASHINGTON, DC, USA; APRIL 01 -05, 2006 ISSN: 0197-016X
- LI QING ET AL: "Concordant methylation of the ER and N33 genes in glioblastoma multiforme" ONCOGENE, Bd. 16, Nr. 24, 18. Juni 1998 (1998-06-18), Seiten 3197-3202, XP002449274 ISSN: 0950-9232
- ZEMLIAKOVA V V ET AL: "[Abnormal methylation of several tumor suppressor genes in sporadic breast cancer]" MOLEKULIARNAIA BIOLOGIIA 2003 JUL-AUG, Bd. 37, Nr. 4, Juli 2003 (2003-07), Seiten 696-703, XP009088991 ISSN: 0026-8984
- KATSAROS D ET AL: "Methylation of tumor suppressor gene p16 and prognosis of epithelial ovarian cancer" GYNECOLOGIC ONCOLOGY, ACADEMIC PRESS, LONDON, GB, Bd. 94, Nr. 3, September 2004 (2004-09), Seiten 685-692, XP004537049 ISSN: 0090-8258
- KWONG J ET AL.: "DLEC1, a Candidate Tumor Suppressor Gene on 3p21.3, is Frequently Silenced in Human Epithelial Ovarian Cancer." GYNECOLOGIC ONCOLOGY. ABSTRACTS PRESENTED FOR THE THIRTY-SIXTH SOCIETY OF GYNECOLOGIC ONCOLOGISTS MEETING., Bd. 96, Nr. 3, März 2005 (2005-03), Seiten 939-940, XP002449275 ISSN: 0090-8258
- WEI SUSAN H ET AL: "Methylation microarray analysis of late-stage ovarian carcinomas distinguishes progression-free survival in patients and identifies candidate epigenetic markers." CLINICAL CANCER RESEARCH : AN OFFICIAL JOURNAL OF THE AMERICAN ASSOCIATION FOR CANCER RESEARCH JUL 2002, Bd. 8, Nr. 7, Juli 2002 (2002-07), Seiten 2246-2252, XP002449289 ISSN: 1078-0432
- XU X L ET AL: "Methylation profile of the promoter CpG islands of 31 genes that may contribute to colerectal carcinogenesis", WORLD JOURNAL OF GASTROENTEROLOGY, WJG PRESS, CN, vol. 10, 1 January 2004 (2004-01-01), pages 3441-3454, ISSN: 1007-9327

## Beschreibung

Die Erfindung betrifft ein Verfahren und die Verwendung von PCR-Primern für die Diagnose bei Eierstockkrebs und/oder die Prognogestellung bei Eierstockkrebs. Ebenfalls beschrieben ist ein Verfahren zur Abschätzung der Wirksamkeit von Therapeutika bei der und ein Verfahren zur Therapieplanung für die Behandlung von Eierstockkrebs.

Bösartiger Eierstockkrebs ist die letalste gynäkologische Krebserkrankung und mit etwa 6% die vierthäufigste Todesursache bei krebskranken Frauen in westlichen Ländern. Schätzungen geben an, dass eine von 70 Frauen in ihrer Lebenszeit an Eierstockkrebs erkrankt, mit einer mittleren Überlebensrate von 4,5 Jahren. Eine neuere Krebsstatistik gibt geschätzte 22.200 neue Fälle und 16.210 Tote pro Jahr in den Vereinigten Staaten an (Jemal A, Murray T, Ward E, Samuels A. Cancer statistics, 2005 (vol 55, pg 10, 2005). Ca-A Cancer Journal for Clinicians 2005; 55(4):259).

5% bis 10% aller Eierstockkrebspatientinnen haben eine erbliche Prädisposition und leiden hauptsächlich am sogenannten "Erblichen Brust/Eierstockkrebssyndrom" oder am "Lynch II-Syndrom". Die meisten Fälle sind aber sporadische Eierstockkrebserkrankungen. Bei genetisch bedingten Hochrisikopatientinnen reduzieren prophylaktische Ovariektomien das Risiko und intensiviertes Screenen kann frühere Diagnosen in der Zukunft erlauben. In der allgemeinen Bevölkerung stellt die frühere Diagnose eine Hauptaufgabe dar, denn mehr als drei Viertel der Fälle werden in einem späteren Stadium (FIGO III und IV) diagnostiziert. Alle diese Patientinnen benötigen eine multimodale Therapie, bestehend aus einer optimalen Debulking-Operation und systemischer Chemotherapie. Molekularmarker, die mit dem Behandlungsausgang korrelieren und daher Behandlungsentscheidungen unterstützen sind erforderlich.

Derzeit anerkannte prognostische Marker beinhalten die anfängliche Stadieneinteilung, Grading und Größe der verbleibenden nicht entfernbaren Tumormasse nach der zytoreduktiven Operation. (van der Burg ME. Advanced ovarian cancer. Curr Treat Options Oncol 2001; 2(2):109-118; Holschneider CH, Berek JS. Ovarian cancer: Epidemiology, biology, and prognostic factors. Seminars in Surgical Oncology 2000; 19(1):3-10). Molekularmarker für die frühe Auffindung und Prognose neben dem Tumormarker "Cancer Antigen 125" (CA-125) (Makar AP, Kristensen GB, Kaern J, Bormer OP, Abeler VM, Trope CG. Prognostic value of pre- and postoperative serum CA 125 levels in ovarian cancer: new aspects and multivariate analysis. Obstet Gynecol 1992; 79(6):1002-1010) existieren in der klinischen Praxis nicht (Ludwig JA, Weinstein JN. Biomarkers in Cancer Staging, Prognosis and Treatment Selection Nat Rev Cancer 2005).

Aufgabe der vorliegenden Erfindung ist die Diagnose und die Prognosesstellung von Eierstockkrebs, sowie die Schaffung eines Diagnose- und Prognosestellungsverfahrens für Eierstockkrebs und eines Testkits dafür sowie die Schaffung eines Verfahren zur Abschätzung der Wirksamkeit von Therapeutika bei der und eines Verfahren zur Therapieplanung für die Behandlung von Eierstockkrebs.

Die Erfinder der vorliegenden Erfindung haben herausgefunden, dass *TUSC3* als Molekularmarker bei Eierstockkrebs geeignet ist. Die Messung der Expression des Markers *TUSC3* in einer biologischen Probe, vorzugsweise einer Gewebeprobe, und das Vergleichen mit der Expression des Markers in biologischen Proben gesunder Frauen kann zur Diagnose von Eierstockkrebs beitragen. Ein entsprechendes Verfahren kann die folgenden Schritte umfassen:
- Isolation der RNA aus der zu untersuchenden biologischen Probe, vorzugsweise dem zu untersuchenden Gewebe,
- Herstellung von cDNA mittels der isolierten RNA,
- Durchführung einer Echtzeit-PCR für den Marker *TUSC3,*
- Vergleich des Expressionslevels des Markers aus der zu untersuchenden biologischen Probe mit dem Level in biologischen Proben gesunder Frauen.

*TUSC3*, ursprünglich *N33* genannt, wurde als ein potenzielles Tumorsuppressorgen aus dem Chromosomenabschnitt 8p22 in Prostatakrebs in der Mitte der 90er Jahre identifiziert (Bova GS, MacGrogan D, Levy A, Pin SS, Bookstein R, Isaacs WB. Physical mapping of chromosome 8p22 markers and their homozygous deletion in a metastatic prostate cancer. Genomics 1996; 35(1):46-54; MacGrogan D, Levy A, Bova GS, Isaacs WB, Bookstein R. Structure and methylation-associated silencing of a gene within a homozygously deleted region of human chromosome band 8p22. Genomics 1996; 35(1):55-65). Die Basis für diese Hypothese war die Identifizierung von mehreren homozygoten Deletionen auf dem Chromosomenabschnitt 8p22 in Prostatakrebszellen. Die Proteinvorhersage ergab, dass das kodierte Protein eine mutmaßliche Untereinheit des Oligosaccharyltransferase-Komplexes ist (Kelleher DJ, Karaoglu D, Mandon EC, Gilmore R. Oligosaccharyltransferase isoforms that contain different catalytic STT3 subunits have distinct enzymatic properties. Molecular Cell 2003; 12(1):101-111). *TUSC3* wurde in einigen Krebszelllinien homozygot deletiert, aber selten mutiert gefunden. Mutationsanalysen, die bei Prostatakrebs und anderen Tumorformen durchgeführt wurden, die häufig Verlust der Heterozygotie (LOH) und/oder homozygote Deletionen auf 8p22 zeigen, zeigten keine signifikante Rate an inaktivierenden Mutationen. Missensmutationen waren aufgrund des Fehlens bekannter Proteinfunktionen schwer zu interpretieren und das Interesse an *TUSC3* und seinem kodierten Protein verschwand daher.

Einsicht über Haploinsuffizienz und epigenetische Modifikationen von Tumorsuppressorgenen haben das Interesse an alten Kandidatenmarkern wieder geweckt. Kürzlich verfügbar gemachte Datenbanken und In-Silico-Tools unterstützen diese Bemühungen.

Unter Verwendung öffentlich erhältlicher Genexpressionsdaten, haben die Erfinder systematisch nach reduzierter Expression von Genen auf 8p22 (nicht veröffentlichte Daten) gesucht und *TUSC3* (*N33*) als eines der Gene identifiziert, deren Expression signifikant bei Eierstockkrebs herabgesetzt ist. Eine quantitative Expressionsanalyse durch Real-Time RT-PCR bestätigte die signifikante Herabsetzung von *TUSC3* in Eierstockkrebsgeweben. Die statistische Analyse der Korrelation der *TUSC3-*Expression mit klinischen Parametern zeigte, dass Patientinnen mit reduzierter *TUSC3*-Expression zum Zeitpunkt der Diagnose ein kürzeres Gesamtüberleben verglichen mit Patientinnen haben, deren *TUSC3* Niveaus in ihren Tumoren gleich jenen in ihren normalen Eierstockgeweben waren. Diesen Ergebnissen wurde nachgegangen und an einer großen unabhängigen Patientenpopulation nochmals getestet. Die anfänglichen Beobachtungen einer möglichen prognostischen Bedeutung der *TUSC3*-Expression konnten bestätigt werden.

Die Erfindung ist in den Ansprüchen definiert.

Die Erfinder der vorliegenden Erfindung haben herausgefunden, dass *TUSC3* auch als Molekularmarker bei der Diagnose von und/oder Prognosestellung bei Eierstockkrebs, sowie zur Abschätzung der Wirksamkeit einer platinbasierenden Chemotherapie und/oder einer Therapie auf Basis von DNA-Demethylierungsagenzien, wie Decitabin (5-Aza-2'-deoxycytidin) oder Zebularin, und/oder auf Basis von Inhibitoren der Histon-Deacetylasen bei Eierstockkrebs dienen kann, indemdie Promotor-Hypermethylierung des Markers *TUSC3* in einer biologischen Probe, vorzugsweise in einer Gewebeprobe oder biologischen Flüssigkeit, wie Vollblut, Serum oder Plasma, gemessen wird. Vorzugsweise umfasst das Verfahren folgende Schritte:
- Isolation der DNA aus der biologischen Probe, vorzugsweise der Gewebeprobe oder biologischen Flüssigkeit
- Durchführung eines PCR-basierenden Methylierungsnachweises
- Analyse der gewonnenen PCR-Produkte.

Vorteilhafterweise erfolgt der PCR-basierende Methylierungsnachweis mit methylierungsspezifischer PCR. Dabei kann für den Nachweis methylierter DNA eine PCR z.B. mit einem der folgenden Primerpaare durchgeführt werden:
- 5'-TTTTCGGTGAATCGGATG-3' und
   5'-TACGCGCCCAACTCCTA-3',
- 5'-GGTTTAGTTAGCGGGTTTTCGG-3' und
   5'-AAACAAATACCGCAACCGCCG-3' oder
- 5'-GGTCGGGTAGGCGTGGTGCG-3' und
   5'-CCGCCCCGCTTACCTACGACGT-3'.

Zusätzlich kann für den Nachweis nicht methylierter DNA eine PCR mit einem der folgenden Primerpaare durchgeführt werden:
- 5'-GGGTTTAGTTAGTGGGTTTTfGGAG-3' und
   5'-AAACAAATACCACAACCACCACCC-3' oder
- 5'-GAGGTTGGTTGGGTAGGTGTGGTGTG-3' und
   5'-CACAACCACCACCCCACTTACCTACAACAT-3'.

Alternativ kann der PCR-basierende Methylierungsnachweis mit methylsensitiver Restriktionsendonuklease-Reaktion und PCR erfolgen.

Dazu kann z.B. das folgende Verfahren verwendet werden:

Zum spezifischen Nachweis methylierter Sequenzen werden 500 ng einer genomischen DNA mit 40 Units des Restriktionsenzyms Hpa II (New England Biolabs, Ipswich, Ma, USA), unter Verwendung des Reaktionspuffers NEBuffer 1 (New England Biolabs, Ipswich, Ma, USA), in einem Volumen von 50 µl gemischt mit Mineralöl überschichtet und 18 Std. bei 37 °C behandelt. Darauffolgend wird die verdaute DNA mit dem Invisorb Spin PCRapid Kit (Invitek, Berlin, Deutschland) gereinigt und in einem Volumen von 50 µl aufgenommen. Der PCR-Ansatz umfasst 25 µl und setzte sich aus je 0.3 µM der beiden biotinilierten Primer (vorwärts: 5'-bio-GCCTTTCCAGGTCTTCTCC-3'; rückwärts: 5'-bio-CCACGCGCGGTAGCCGTG-3'), 50 ng der verdauten und gereinigten DNA, 1 Unit SuperTaq (HT Biotech, Cambridge, UK), 1 x SuperTaq Puffer (HT Biotech, Cambridge, UK) und 0,2 mM der dNTPs (Larova, Teltow, Deutschland) zusammen. Die PCR wird unter folgenden Bedingungen durchgeführt: 2 Min. bei 94 °C, 40 Zyklen - 15 Sek. bei 94 °C, 30 Sek. bei 58 °C, 30 Sek. bei 72 °C - und schließlich 3 Min. bei 72 °C. Der Nachweis entstandener PCR-Produkte erfolgt auf 2 % Agarose-Gelen und Ethidiumbromid-Färbung. Darüberhinaus ermöglicht die Verwendung biotinilierter Primer eine Analyse der PCR-Produkte auch durch alternative Methoden z. B. sequenz-spezifisches Capturing im Zuge der reversen Hybridisierung und der anschließenden Detektion der gebundenen biotinilierten Sequenzen mittels Streptavidin-Enzym-Konjugat.

Ein ebenfalls hier beschriebener Testkit zur Diagnose von und/oder Prognosestellung bei Eierstockkrebs und/oder Abschätzung der Wirksamkeit einer platinbasierenden Chemotherapie und/oder einer Therapie auf Basis von DNA-Demethylierungsagenzien und/oder auf Basis von Inhibitoren der Histon-Deacetylasen bei Eierstockkrebs enthält PCR-Primer, die für die Durchführung einer methylierungsspezifischen PCR in der *TUSC3*-Promotorregion geeignet sind.

Alternativ kann der Testkit ein Restriktionsenzym und PCR-Primer, die die entsprechende Restriktionsschnittstelle innerhalb der TUSC3-Promotorregion flankieren, enthalten.

Zusätzlich kann der Testkit Mittel zum Nachweis entstandener PCR-Produkte, wie eine Hybridisierungssonde, enthalten.

Beim ebenfalls hier beschriebenen Verfahren zur Therapieplanung für die Behandlung von Eierstockkrebs, wird ein Verfahren zur Abschätzung der Wirksamkeit einer platinbasierenden Chemotherapie und/oder einer Therapie auf Basis von DNA-Demethylierungsagenzien, wie Decitabin (5-Aza-2'deoxycytidin) oder Zebularin, und/oder auf Basis von Inhibitoren der Histon-Deacetylasen bei Eierstockkrebs durchgeführt, bei dem die Promotor-Hypermethylierung des Markers TUSC3 in einer zu untersuchenden biologischen Probe, vorzugsweise in einer zu untersuchenden Gewebeprobe oder biologischen Flüssigkeit gemessen wird und bei Feststellung einer Hypermethylierung des *TUSC3*-Promotors wird eine Therapie auf Basis von DNA-Demethylierungsagenzien, wie Decitabin (5-Aza-2'-deoxycytidin) oder Zebularin, und/oder auf Basis von Inhibitoren der Histon-Deacetylasen vorgeschlagen.

Bei einem Verfahren zur Therapieplanung für die Behandlung von Eierstockkrebs, wird ein Verfahren zur Abschätzung der Wirksamkeit einer platinbasierenden Chemotherapie und/oder einer Therapie auf Basis von DNA-Demethylierungsagenzien, wie Decitabin (5-Aza-2'-deoxycytidin) oder Zebularin, und/oder auf Basis von Inhibitoren der Histon-Deacetylasen bei Eierstockkrebs durchgeführt indem die Promotor-Hypermethylierung des Markers TUSC3 in einer zu untersuchenden biologischen Probe, vorzugsweise in einer zu untersuchenden Gewebeprobe oder biologischen Flüssigkeit gemessen wird, und es wird eine platinbasierende Chemotherapie vorgeschlagen, wenn keine Hypermethylierung oder des *TUSC3*-Promotors festgestellt wird.

Promotor-Hypermethylierung von Tumorsuppressorgenen (TSGs) ist als Molekularmarker vielversprechend. Es kann Einblick in die Biologie eines Tumors geben, und kann im Gegensatz zur Gen- oder Proteinexpression leicht durch auf PCR basierende Methoden nicht nur aus dem Tumormaterial sondern auch in Körperflüssigkeiten nachgewiesen werden (Das PM, Singal R. DNA methylation and cancer. J Clin Oncol 2004; 22(22):4632-4642; Jones PA, Baylin SB. The fundamental role of epigenetic events in cancer. Nat Rev Genet 2002; 3(6):415-428; Laird PW. The power and the promise of DNA methylation markers. Nat Rev Cancer 2003; 3(4):253-266).

Hypermethylierung des *TUSC3* beinhaltenden Chromosomenabschnitts wurde in Prostata-, Dickdarm-, Leber-, Lungen- und Blasenkrebs untersucht (MacGrogan D, Levy A, Bova GS, Isaacs WB, Bookstein R. Structure and methylation-associated silencing of a gene within a homozygously deleted region of human chromosome band 8p22. Genomics 1996; 35(1):55-65. Ahuja N, Li Q, Mohan AL, Baylin SB, Issa JP. Aging and DNA methylation in colorectal mucosa and cancer. Cancer. Res 1998; 58(23):5489-5494.). Altersabhängige Hypermethylierung wurde nur in normalem und neoplastischem Dickdarmgewebe, nicht aber bei anderen Krebsarten gefunden. Vor kurzem hat eine andere Gruppe keine Hypermethylierung von *TUDC3* in 65 untersuchten Dickdarmkrebsproben gefunden (Xu XL, Yu J, Zhang HY et al. Methylation profile of the promoter CpG islands of 31 genes that may contribute to colorectal carcinogenesis. World J Gastroenterol 2004; 10(23):3441-3454).

Hypermethylierung wurde kürzlich in einem breiten Bereich von krebsbezogenen Genen in Eierstockkrebs untersucht. Die Auswahl der Gene basierte hauptsächlich auf Zufälligkeit und Erhältlichkeit etablierter Nachweissysteme basierend auf methylierungsspezifischer PCR (Makarla PB, Saboorian MH, Ashfaq R et al. Promoter hypermethylation profile of ovarian epithelial neoplasms. Clin Cancer Res 2005; 11(15):5365-5369; Teodoridis JM, Hall J, Marsh S et al. CpG Island Methylation of DNA Damage Response Genes in Advanced Ovarian Cancer. Cancer Res 2005; 65(19):8961-8967). Obwohl diese Ergebnisse für die Entwicklung von Screeningtests für Krebserkrankungen im Allgemeinen wertvoll sind, können Promotorhypermethylierungen relativ späte und unspezifische Ereignisse in der Karzinogenese darstellen. Ein idealer Marker würde auch Hinweise auf das biologische Verhalten geben. Kürzlich konnte *p15^{INK4b}* nach Korrektur in Bezug auf etablierte prognostische Faktoren in einem multivariaten Cox Regressionsmodel (Liu Z, Wang LE, Wang L et al. Methylation and Messenger RNA Expression of p15INK4b but Not p16INK4a Are Independent Risk Factors for Ovarian Cancer. Clin Cancer Res 2005; 11(13):4968-4976) als Risikofaktor für Eierstockkrebs identifiziert werden.

Die Erfinder haben festgestellt, dass der Prozentsatz der Patientinnen, die gegen Platin-basierende Chemotherapie resistent waren, in der Gruppe mit hypermethylierten *TUSC3*-Promotoren signifikant höher war, was eine bestimmte notwendige Expressionsstärke von *TUSC3* für ein günstiges Ansprechen auf Chemotherapie impliziert.

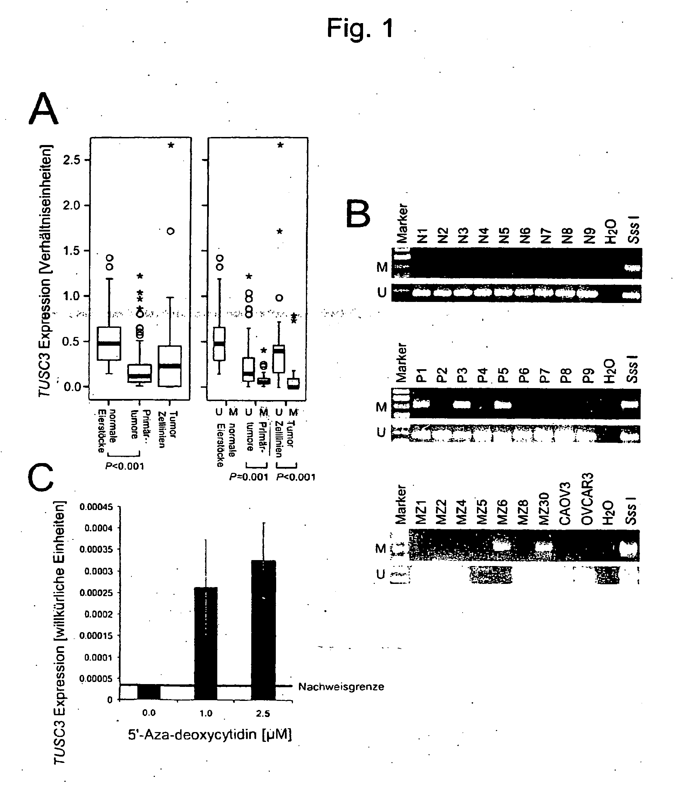

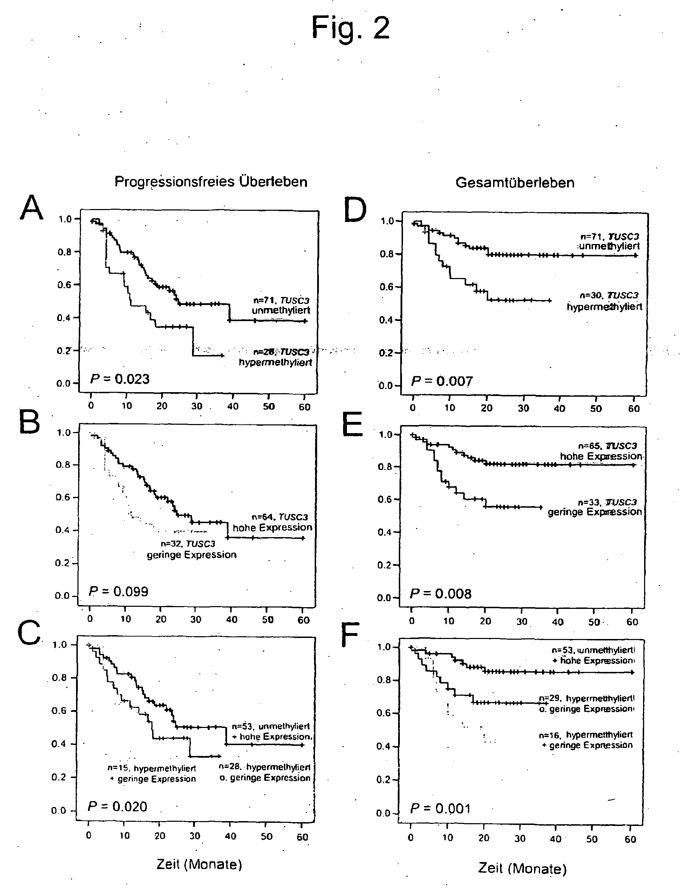

Im Folgenden sollen Untersuchungen, die die Erkenntnisse stützen, auf denen die vorliegende Erfindung basiert, beschrieben werden. Dabei wird auch auf die beiliegenden Zeichnungen Bezug genommen, in denen Fig.1A Boxplots der *TUSC3-*Gesamtexpression bzw. der Expression der nicht methylierten und hypermethylierten Fraktion von gutartigen Eierstockgewebeproben, Eierstockkrebsgewebeproben und Eierstockkrebszelllinen zeigt, Fig.1B Ethidiumbromide-Agarosegel-Fotos von methylierungsspezifischen PCR-Produkten von gutartigen Eierstockgewebeproben, Eierstockkrebsgewebeproben und Eierstockkrebszelllinien zeigt, Fig. 1C die Wiederherstellung von *TUSC3*-Expression in Eierstockkrebszelllinien mit 5-aza-deoxycytidin zeigt und Fig.2 von Kaplan-Meier-Plots für progressionsfreies und Gesamtüberleben von Patientinnen, deren Tumorproben unterschiedliche Expressionsintensitäten und Methylierungszustände zeigten, zeigt.

### UNTERSUCHUNGEN

### Patientenmaterial und Krebszelllinien

Tumorproben wurden von Eierstockkrebspatientinnen zum Zeitpunkt der Primärdiagnose von Eierstockkrebs erhalten, die sich einer Operation unterzogen hatten. Epithelial angereicherte normale Eierstockproben und Proben gutartiger Zysten kamen von Patientinnen, die ohne maligne Erkrankung diagnostiziert wurden. Insgesamt wurden 20 gutartige Eierstockproben und 102 Primärtumorproben von 102 Patientinnen beurteilt. Bei den Tumorproben wurde, wie für eine repräsentative Eierstockkrebspopulation erwartet, eine Mehrheit der Patientinnen (72%) mit fortgeschrittenen Stadien (III und IV) diagnostiziert. 84% der Patientinnen wurden nach einem auf Platin basierenden Chemotherapie-Schema behandelt. Die entsprechenden Daten sind in Tabelle 1 zusammengefasst.

| **Tabelle 1. Klinisch-pathologische Merkmale von Eierstockkrebspatientinnen** | |
|---|---|
| **A) Gutartige Eierstockproben (entsprechende mRNA-Proben)** | 20 (20) |
| Alter bei Operation [Jahren] | |
| Mittel ± Standardabweichung | 49.9 ± 11.8 |
| 7 normale Eierstöcke, mittleres Alter [Jahre]: | 51.6 |
| 13 gutartige Zysten, mittleres Alter [Jahre]: | 49.0 |

| **B) Tumorproben (entsprechende mRNA-Proben)** | 102 (99) |
|---|---|
| Alter bei Diagnose [Jahre] | |
| Mittel ± Standardabweichung | 58.3 ± 11.0 |

| Histoloqie (1 (1.0%) fehlend) | |
|---|---|
| serös | 77 (75.5%∞) |
| endometrioid | 10 (9.8%) |
| muzinös | 5 (4.9%) |
| klarzellig | 2 (2.0%) |
| andere | 7 (6.9%) |

| FIGO (2(2.0%) fehlend) | |
|---|---|
| I | 17 (16.7%) |
| II | 10 (9.8%) |
| III | 51 (50.0%) |
| IV | 22 (21.6%) |

| Grading (1 (1.0%) fehlend) | |
|---|---|
| 1 | 2 (2.0%) |
| 2 | 48 (46.1%) |
| 3 | 51 (50.0%) |

| Chemotherapie (3 (2.9%) fehlend) | |
|---|---|
| Carboplatin und Taxol | 63 (61.8%) |
| Carboplatin, Taxol und Gemcitabin | 17 (16.7%) |
| Carboplatin oder Taxol Monotherapie | 3 (2.9%) |
| Carboplatin und andere | 8 (7.8%) |
| andere | 3 (2.9%) |
| keine | 5 (4.9%) |

| Ansprechen auf Chemotherapie (16 (15.7%) fehlend) | |
|---|---|
| pNED‡ | 6 (5.9%) |
| pNC† | 3 (2.9%) |
| pPD† | 12 (11.8%) |
| pPR† | 5 (4.9%) |
| pCR‡ | 43 (42.2%) |

| Dauer des Ansprechens | |
|---|---|
| 0 - 6 Monate†,‡ | 6 (5.9%) |
| 6 - 12 Monate‡ | 5 (4.9%) |
| > 12 Monate‡ | 6 (5.9%) |

| Remissionen/progressive Erkrankunq (3 (2.9%) fehlend) | |
|---|---|
| ohne Remission | 49 (48.0%) |
| mit Remission | 33 (32.4%) |
| progressive Erkrankung | 17 (16.7%) |
| ^{∞}Prozentsatz basierend auf 102 Tumorproben. | |
| ^{†}Nichtanprechende auf Chemotherapie. | |
| ^{‡}Ansprechende auf Chemotherapie. | |

Zusätzlich wurden 32 Eierstockkrebszelllinien untersucht, die aus Eierstockkrebsgewebeproben von 28 Patentinnen angelegt und wie beschrieben kultiviert wurden (Mobus V, Gerharz CD, Press U, et al., Morphological, immunohistochemical and biochemical characterization of 6 newly established human ovarian carcinoma cell lines. Int J Cancer.1992; 52:76-84).

### DNA und RNA Isolation

Genomische DNA von etwa 15 mg gefrorenem Gewebe wurde mit dem DNeasy Tissue Kit (Qiagen, Hilden, Deutschland) wie vom Hersteller beschrieben, isoliert. Die DNA-Menge wurde unter Verwendung des PicoGreen dsDNA Quantitation Kit (Molecular Probes, Inc., Eugene, OR, USA) gemessen und die DNA-Qualität wurde stichprobenartig mit 0,3% Agarose-Gel-Elektrophorese bewertet. DNA aus Eierstockkrebszelllinien wurde unter Verwendung des FlexiGen DNA Kit (Qiagen, Hilden, Deutschland) isoliert.

Gesamt-RNA aus gefrorenen Geweben wurde mit dem Agilent Total RNA Isolation Mini Kit (Agilent Technologies, Palo Alto, CA, USA) gemäß den Angaben des Herstellers, extrahiert. Gesamt-RNA aus Krebszelllinien wurde mit dem RNeasy Mini Kit (Qiagen, Hilden, Deutschland) isoliert. RNA Qualität und Quantität wurden mittels Kapillarelektrophorese unter Verwendung von RNA Nano Chips (Lab-on-a-Chip, Agilent Technologies, Palo Alto, CA, USA) beurteilt.

### Methylierungsspezifische PCR (MSP) und DNA-Demethylierung

Zur Bisulfit-Behandlung wurde 1 µg genomische DNA mit den Restriktionsenzymen *Hind* III und *Xba* I verdaut, präzipitiert, gelöst und für 20 Minuten in 0,35 M NaOH denaturiert. Danach wurde die DNA in 8 Zyklen (2 Std. 75°C und 1 Min. 95°C) mit 1,75 M Natiumbisulfit, 5,23 M Harnstoff und 0,52 mM Hydroquinon in einem PCR-Cycler behandelt. Die DNA wurde unter Verwendung des QIAEX II Gel Extraction Kit (Qiagen, Hilden, Deutschland) gereinigt, und die Reaktion wurde mit 0,32 M NaOH (20 Min. 37°C) beendet. Schließlich wurde die DNA mit 1 µl Glycogen als Träger NH₄Acetat-EtOH-präzipitiert mit 70% EtOH gewaschen und in 40 µl H₂O gelöst. Für die methylierungsspezifische Reaktion wurde PCR mit 25 ng Bisulfit behandelter DNA, Primern die von Xu et al. publiziert worden waren (Xu XL, Yu J, Zhang HY et al. Methylation profile of the promoter CpG islands of 31 genes that may contribute to colorectal carcinogenesis. World J Gastroenterol 2004; 10(23):3441-3454), AmpliTaq Gold (Applied Biosystems, Foster City, CA, USA), 1x Optimized Buffer B (Invitrogen, Carlsbad, CA, USA) unter den folgenden PCR Bedingungen durchgeführt: 10 Min. bei 94°C, 50 Zyklen von 30 Sek. bei 94°C - 30 Sek. bei 50°C - 20 plus 1 Sek. pro Zyklus bei 72°C und schließlich 7 Min. bei 72°C. Für den Nachweis nicht methylierter DNA wurde PCR mit den folgenden Primern (sense: 5'-GAGGTTGGTTGGGTAGGTGTGGTGTG-3' und antisense: 5'-CACAACCACCACCCCACTTACCTACAACAT-3') und ähnlichen PCR-Bedingungen wie oben durchgeführt (außer: 1x Optimized Buffer F (Invitrogen, Carlsbad, CA, USA) und 56°C Annealing Temperatur). Die PCR-Produkte wurden auf 3% Agarose-Gelen, gefärbt mit Ethidiumbromid analysiert. Bisulfitsequenzieren wurde direkt mit den PCR-Produkten, die mit dem QIAquick PCR Purification Kit (Quiagen, Hilden, Deutschland) gereinigt wurden, durchgeführt. DNA-Demethylierung der Krebszelllinien wurde durch eine 72 Stunden Behandlung mit 1 und 2,5 µM 5-Aza-Deoxycytidin (Sigma-Aldrich, St. Louis, MO, USA) in Zellkulturmedium durchgeführt und die relative *TUSC3* Expression wurde durch Real-Time RT-PCR wie unten beschrieben bewertet.

### cDNA Synthese und quantitative Real-Time RT-PCR

cDNA wurde von 1 µg DNase I-verdauter Gesamt-RNA unter Verwendung des DuraScript RT-PCR Kits (Sigma-Aldrich, Saint Louis, MO, USA) und einer 1:1-Mischung des zur Verfügung gestellten Oligo-(dT)₂₃ und Random-Nonamer Oligo, im Wesentlichen wie im Handbuch beschrieben, synthetisiert. TaqMan Real-Time PCR wurde durchgeführt und die relative Expression (verglichen mit einem Kalibrator, einer cDNA-Mischung aus mehreren Zelllinien) wurde aus den Schwellenwert-Zyklen (Ct), die mit der GeneAmp 5700 SDS v1.3 (Applied Biosystems) wie folgt errechnet: 2^-[(Ct_{gene} Mittel von duplizierten Sonden - Ct_{gene} Mittel von dupliziertem Kalibrator) - (Ct_{B2M} Mittel von duplizierten Sonden - Ct_{B2M} Mittel von dupliziertem Kalibrator)] (und als willkürliche "Verhältniseinheiten" angegeben) wobei die folgenden Assay-on-Demand^{™} Sonden verwendet wurden: *TUSC3*, Hs00185147_ m1 und *beta* 2-*microglobulin,* Hs99999907_m1 (Applied Biosystems, Foster City, CA, USA).

### Statistische Analyse

Kontinuierliche Variable sind als Mittelwert und Standardabweichung dargestellt, Kategorievariable als absolute und relative Frequenzen. Um Frequenzen zwischen zwei oder mehreren Gruppen zu vergleichen, wurde ein Fisher-Exakt-Test bzw. ein Fisher-Freeman-Halton-Test jeweils durchgeführt.

Im Falle einer ausreichenden Annäherung an eine Normalverteilung wurden Mittelwerte und 95% Vertrauensintervalle für *TUSC3* Expression in einem logarithmischen Maßstab berechnet und dann in den Originalmaßstab zurücktransformiert. Um die *TUSC3* Expression zwischen zwei oder mehr Gruppen zu vergleichen, wurde ein t-Test bzw. ein one-way ANOVA durchgeführt, und zwar unter Verwendung der logtransformierten Expression als unabhängige Variable. Analog wurde der mögliche Einfluss von Hypermethylierung auf *TUSC3* Expression unter Verwendung eines t-Tests im logarithmischen Maßstab untersucht.

Der mögliche Einfluss der *TUSC3* Expression und von Hypermethylierung auf das progressionsfreie und Gesamtüberleben ist in entsprechenden Kaplan-Meier-Plots dargestellt, und unter Verwendung von Cox Proportional Hazard-Regressionsmodellen quantifiziert. Zu diesem Zweck wurde die Expression an der 33,33 % Perzentile dichotomisiert, da ungefähr 30% der Proben hypermethyliert waren und eine starke Korrelation von Hypermethylierungszustand und Expression beobachtet wurde; ferner wurde eine Kombination dieser dichotomen Variablen und des Hypermethylierungsstatus in drei Niveaus (3 = geringe Expression und hypermethyliert, 2 = entweder geringe Expression oder hypermethyliert, 1 = hohe Expression und nicht methyliert) ermittelt und als linearer Faktor in den Cox-Regressionsmodellen berücksichtigt; daher lässt sich das entsprechende Hazard-Verhältnis auf beide Vergleiche von benachbarten Gruppen (3 *vs* 2 und 2 *vs* 1) anwenden. Als Progressionszeitpunkt wurde der Tag der Dokumentation des ersten Rückfalls oder die erste Dokumentation der Progression der Erkrankung, beginnend mit dem Zeitpunkt der ersten Diagnose, verwendet.

Univariate Coxmodelle wurden verwendet, um den Einfluss bekannter prognostischer Faktoren und der drei möglichen neuen prognostischen Faktoren, die oben beschrieben wurden, zu zeigen. Für jeden der neuen Faktoren wurde ein multivariates Coxmodell, mit bekannten prognostischen Faktoren, als Einstellungsvariable verwendet.

P-Werte ≤0,05 wurden als statistisch signifikant betrachtet. Alle Berechnungen wurden unter Verwendung von SAS Software Version 9.1 (SAS Institute Inc., Cary, NC, USA, 2001) durchgeführt; graphische Darstellungen wurden unter Verwendung von SPSS Software Version 12.0 (SPSS Inc. Headquarters, Chicago, Illinois, USA) hergestellt.

### ERGEBNISSE

### TUSC3 mRNA-Expression ist bei Eierstockkrebs reduziert

Quantitative Real-Time RT-PCR von 99 Eierstockkrebsgeweben und 20 gutartigen Eierstockgeweben wurde durchgeführt. Die Fig. 1A zeigt Boxplots der Expression von TUSC3 in beliebigen Einheiten (Verhältniseinheiten) basierend auf der Expression von ß-2-Mikroglobulin als interne Referenz (Housekeepinggenkontrolle) von gutartigen Eierstockgewebeproben (normale Eierstöcke), Eierstockkrebsgewebeproben (Primärtumore) und Eierstockkrebszelllinen (Tumorzelllinien). Im rechten Teil der Grafik wurde die Expression von TUSC3 in nicht methylierte (U) und hypermethylierte (M) Fraktionen unterteilt. Für die statistische Analyse wurde ein t-Test im logarithmischen Maßstab verwendet. Verglichen mit Kontrollen (0,465, CI 0,347-0,623 Verhältniseinheiten) war die Expression in Tumoren signifikant (P < 0,001) niedriger (0,105, CI 0,082-0,135 Verhältniseinheiten). Signifikante Unterschiede in der *TUSC3* Expression zwischen Tumorproben von Patientinnen verschiedener Altersgruppen (≤50 und >50), Proben von verschiedenen FIGO-Stadien und Proben unterschiedlicher Differenzierungsgrade (Grading) wurden nicht gefunden (Tabelle 2A). Nur die Expression in nichtserösen Tumoren war signifikant geringer (P = 0,037, nicht korrigiert für mehrfaches Testen) verglichen mit serösen Tumoren (Tabelle 2A).

| **Tabelle 2.** Vergleich der klinisch-pathologischen Merkmale mit *TUSC3* Expression und Hypermethylierung | | |
|---|---|---|
| **A) *TUSC3* Expression** | | |
| Klinisch-pathologische Merkmale | Expression von *TUSC3* | Vergleich (*P*) |
| Gutartige Eierstockproben | | |
| Median | 0.465 | |
| CI | 0.347-0.623 | |
| | | |
| Tumorproben (Vergleich der gutartigen Eierstockproben) | | <0.001^{†} |
| Median | 0.105 | |
| CI | 0.082-0.135 | |
| Alter | | |
| ≤50 | 0.085 | 0.299^{†} |
| > 50 | 0.114 | |
| Histologie | | |
| serös | 0.121 | 0.037^{†} |
| nicht-serös | 0.064 | |
| FIGO | | |
| I | 0.084 | 0.764^{‡} |
| II | 0.124 | |
| III | 0.104 | |
| IV | 0.126 | |
| Grading | | |
| 1 | 0.068 | 0.188^{‡} |
| 2 | 0.135 | |
| 3 | 0.086 | |

| **B) *TUSC3* Hypermethylierung** | | |
|---|---|---|
| Klinisch-pathologische Merkmale | Hypermethylierung von *TUSC3* | Vergleich (P)^{#} |
| Gutartige Eierstockproben | | |
| | 0 / 20 (0%) | |

| Primärtumorproben | | |
|---|---|---|
| Alter | 30 / 102 (29.4%) | 0.003 |
| ≤50 | | 0.218 |
| > 50 | 5 / 26 (19.2%) | |
| Histologie | 25 / 75 (33.3%) | |
| serös | | 0.200 |
| nicht-serös | 21 / 79 (26.6%) | |
| FIGO | 9 / 22 (40.9%) | |
| I | | 0.948 |
| II | 5 / 17 (29.4%) | |
| III | 2 / 10 (20.0%) | |
| IV | 15 / 51 (29.4%) | |
| Grading | 7 / 22 (31.8%) | |
| 1 | | 0.102 |
| 2 | 2 / 2 (100%) | |
| 3 | 15 / 48 (31.2%) | |
| ^{‡}ANOVA (berechnet nach logarithmischer Transformation, Mittel und CI sind nach Rücktransformation in den Originalmaßstab angegeben). | 13 / 51 (25.5%) | |
| ^{†} t-Test (wie oben berechnet) | | |
| ^{#} Fisher-Exakt-Test und Fisher-Freeman-Halton-Test, entsprechend. | | |

### Der TUSC3 Promotor ist hypermethyliert und die TUSC3 Expression kann durch DNA-Demethylierung in Eierstockkrebszelllinien wiederhergestellt werden

Die Erfinder haben gezeigt, dass die Expression von *TUSC3* in 5 von 38 Eierstockkrebszelllinien nicht nachweisbar war, und dass in drei weiteren Zelllinien die Expression mehr als 10fach geringer war, als die mittlere Expression aller Zelllinien (hier nicht dargestellt). Unter Verwendung von methylierungsspezifischer PCR (MSP) wurde gefunden, dass der Promotor von *TUSC3* in sieben von diesen acht Zelllinien mit geringer oder fehlender *TUSC3* Expression hypermethyliert war. Die Fig. 1B zeigt beispielhafte Ethidiumbromid-Agarosegel-Fotos von methylierungsspezifischen PCR-Produkten von neun gutartigen Eierstockgewebeproben (N1-N9), neun Eierstocktumorproben (P1-P9) und neun Eierstockkrebszelllinien (MZ1-OVCAR3). Als positive Kontrolle wurde eine Sss I (CpG-Methylase) methylierte humane DNA und als negative Kontrolle H₂O verwendet. M bezeichnet das methylierte PCR-Produkt und das nicht methylierte PCR-Produkt. Die Expression von *TUSC3* hing signifikant (P < 0,001) vom Hypermethylierungsstatus ab (Fig. 1A).

Eine konzentrationsabhängige (1 und 2,5 µM) Wiederherstellung der *TUSC3* Expression wurde durch eine 72 Stunden Behandlung mit dem demethylierenden Mittel 5-aza-deoxycytidin in der hypermethylierten Zelllinie MZ6 erzielt. In den scheinbehandelten Proben war die TUSC3-Expression unter der Nachweisgrenze. Die TUSC3-Expression war bei Verwendung von 2,5 µM 5-aza-deoxycytidine mindestens 9,5 mal höher. Die Versuche wurden dreifach durchgeführt und die Mittelwerte der relativen TUSC3-Expression (basierend auf der Expression von ß-2-mikroglobulin) mit Standardabweichungen wurden dargestellt.

Um das Methylierungsniveau weiter zu bestimmen und die Spezifität des verwendeten MSP-Systems zu testen, wurde der Methylierungsstatus von einzelnen nahe dem vermeintlichen *TUSC3* Promotor lokalisierten CpG-Inseln durch Bisulfit-Sequenzierung von drei hypermethylierten Zelllinien und von zwei Kontrollen bewertet. Das Sequenzieren der erhaltenen PCR-Produkte zeigte, das fast alle CpGs in den PCR-Produkten der drei Eierstockkrebszelllinien zu mehr als 75%, aber keine CpGs der beiden Kontrollen zu mehr als 10% methyliert waren (Daten nicht gezeigt).

### TUSC3 Hypermethylierung in Eierstocktumorproben

In einem nächsten Schritt wurde die Analyse des Hypermethylierungsstatus des *TUSC3* Promotors durch methylierungsspezifische PCR auf eine Liste von 102 Eierstocktumorproben, die von Patientinnen bei Primärdiagnose erhalten wurden, erweitert (Fig. 1 B). Hypermethylierung von *TUSC3* wurde in 29,8% (30/102) dieser Eierstockkrebsfälle gefunden, aber in keiner von 20 Kontrollen (P = 0,003).

### TUSC3 Hypermethylierung korreliert eng mit TUSC3 Expression

In Fig. 1 ist die *TUSC3* Expression in den hypermethylierten und nichtmetylierten Eierstocktumorgeweben gezeigt. Die mittlere Expression von *TUSC3* in der hypermethylierten Gruppe war signifikant geringer (0,065, CI 0,038-0,081 Verhältniseinheiten) als in der nicht methylierten Gruppe (0,135; CI 0,100-0,182 Verhältniseinheiten, P = 0,001), was eine ähnliche Wechselbeziehung des *TUSC3* Hypermethylierungsstatus und der *TUSC3* Expression, wie sie oben für Eierstockkrebszelllinien gezeigt wurde, bestätigt und einen kausalen Zusammenhang zwischen dem Hypermethylierungsstatus und der Expression in Primärtumorproben nahe legt (Fig. 1A und Tabelle 3).

| **Tabelle 3.** Signifikante Unterschiede von klinisch-pathologischen Merkmalen von hypermethylierten Tumorproben | | | | |
|---|---|---|---|---|
| | | *TUSC3* Hypermethylierung | | |
| Klinisch-pathologische Merkmale | | nicht methyliert | methyliert | Vergleich (*P*) |
| Alter | | n=72 | n=30 | 0.023^{†} |
| | Mittel ± Standardabweichung | 56.7 ± 10.8 | 62.1 ± 10.7 | |
| | Bereich | 35-81 | 41-86 | |
| Expression von *TUSC3* (qRT-PCR) | | n=71 | n=28 | 0.001^{‡} |
| | Mittel | 0.135 | 0.056 | |
| | CI | 0.100-0.182 | 0.038-0.081 | |
| ^{†}t-Test. | | | | |
| ^{‡}t-Test (berechnet nach logarithmischer Transformation, Mittel und CI sind nach Rücktransformation in den Originalmaßstab angegeben). | | | | |

### TUSC3 Hypermethylierung und klinische Merkmale

Es gab keinen signifikanten Unterschied in der Hypermethylierungshäufigkeit zwischen Tumorproben von Patientinnen verschiedener Altersgruppen (≤50 und >50), Proben mit unterschiedlicher histologischer Charakterisierung (serös v nicht-serös), Proben von verschiedenen FIGO-Stadien und Proben von verschiedenen Differenzierungsgraden (Grading) (siehe Tabelle 2B). Interessanterweise war das mittlere Alter in der hypermethylierten Gruppe bei Diagnose um 5,4 Jahre höher (P = 0,023) als in der nicht metylierten Gruppe (Tabelle 3). In der Gruppe der 30 Patienten mit hypermethylierten *TUSC3* Promotoren, hatten mehr Patientinnen eine progressive Erkrankung (32,1%) verglichen mit der Gruppe der 72 Patientinnen ohne *TUSC3* Hypermethylierung (11,3%), was den möglichen Einfluss der *TUSC3* Hypermethylierung und Expression auf die Prognose von Patientinnen mit Eterstockkrebs nahe legt.

### Korrelation des TUSC3 Hypermethylierungsstatus mit progressionsfreiem und Gesamtüberleben

Es wurde ein signifikanter Zusammenhang zwischen der *TUSC3* Hypermethylierung und dem progressionsfreien Überleben (relatives Risiko von 1,972, P = 0,023) und dem Gesamtüberleben (relatives Risiko von 2,915, P = 0,007) gefunden (Fig. 2A und 2B, Tabelle 4). Die Hazardverhältnisse für progressionsfreies Überleben waren vergleichbar mit dem FIGO-Stadium (relatives Risiko von 2,821, P < 0,001) und dem verbleibenden Tumor nach Operation (relatives Risiko von 2,867, P < 0,001) den einzig anderen signifikanten prognostischen Faktoren, die zu dem Zeitpunkt verfügbar sind, wenn Optionen für systemische Therapie sondiert werden müssen.

Das mittlere progressionsfreie Überleben war 11,10 (CI 2,58-19,64) Monate in der hypermethylierten Gruppe verglichen mit 24,55 (CI 11,55-37,55) Monate in der nichtmethylierten Gruppe (Fig. 2A).

Der Zusammenhang zwischen der *TUSC3* Hypermethylierung und der Prognose der Patientinnen war unabhängig von anderen Risikofaktoren einschließlich dem Ansprechen auf systemische Chemotherapie (interaction term nicht signifikant) für das progressionsfreie Überleben (relatives Risiko von 2,024, P = 0.042) und für das Gesamtüberleben (relatives Risiko von 3,817, P = 0,013) (Tabelle 4).

Kaplan-Meier-Schätzungen und Hazardverhältnisse, die mit den dichotomisierten *TUSC3* Expressionsniveaus erhalten wurden, waren ähnlich zu jenen, die unter Verwendung des *TUSC3* Hypermethylierungsstatus erhalten wurden (Fig. 2B und 2E, Tabelle 4). In Fällen, wo der Hypermethylierungsstatus und das Expressionsniveau übereinstimmten, trat der Einfluss auf das progressionsfreie und Gesamtüberleben sogar noch signifikanter hervor (Fig. 2C und 2F, Tabelle 4).

| **Tabelle 4**. Univariate und multivariate Cox - Hazardverhältnisanalyse | | | | |
|---|---|---|---|---|
| | progressionsfreies Überleben | | | |
| | univariat | | multivariat | |
| | Relatives Risiko _{(95% CI)}^{‡} | *P* | Relatives Risiko _{(95% CI)}^{‡} | *P* |
| Alter bei Diagnose | 1.018 _{(0.992-1.045)} | 0.184 | | |
| Histologie (nicht serös *vs* serös)^{†} | 1.156 _{(0.576-2.323)} | 0.683 | | |
| FIGO-Stadium | **2.821** _{(1.867-4.260)} | <0.001 | | |
| Grading | 0.978 _{(0.581-1.646)} | 0.933 | | |
| verbleibender Tumor (ja *vs* nein)^{†} | **2.867** _{(1.615-5.091)} | <0.001 | | |
| Therapie Ansprechen (nein *vs* ja)^{†} | **4.907** _{(2.644-9.104)} | <0.001 | | |
| Methylierung von *TUSC3* (ja *vs* nein) | **1.972** _{(1.099-3.533)} | 0.023 | **2.024** _{(1.025-4.000)} | 0.042 |
| Expression von *TUSC3* (gering *vs* hoch)^{††} | 1.661 _{(0.908-3.304)} | 0.099 | 1.610 _{(0.726-3.571)} | 0.242 |
| ^{L} kombiniert (Methylierung und Expression)^{±} | **1.565** _{(1.072-2.283)} | 0.020 | 1.510 _{(0.915-2.494)} | 0.107 |

| | Gesamtüberleben | | | |
|---|---|---|---|---|
| | univariat | | multivariat | |
| | Relatives Risiko _{(95% CI)}^{‡} | *P* | Relatives Risiko _{(95% CI)}^{‡} | *P* |
| Alter bei Diagnose | **1.056** _{(1.018-1.095)} | 0.003 | | |
| Histologie (nicht-serös *vs* serös)^{†} | **2.369** _{(1.051-5.319)} | 0.037 | | |
| FIGO-Stadium | **2.518** _{(1.411-4.493)} | 0.002 | | |
| Grading | **0.762** _{(0.378-1.534)} | 0.446 | | |
| verbleibender Tumor (ja *vs* nein)^{†} | **5.278** _{(2.193-12.702)} | <0.001 | | |
| Therapie Ansprechen (nein *vs* ja)^{†} | **9.560** _{(3.562-25.654)} | <0.001 | | |
| Methylierung von *TUSC3* (ja *vs* nein) | **2**.**915** _{(1.348-6.289)} | 0.007 | **3.817** _{(1.326-10.989)} | 0.013 |
| Expression von *TUSC3* (gering *vs* hoch)^{††} | **2.985** _{(1.333-6.711)} | 0.008 | 2.079 _{(0.642-6.757)} | 0.222 |
| ^{L} kombiniert (Methylierung und Expression)^{±} | **2.267** _{(1.390-3.690)} | 0.001 | **2.150** _{(1.047-4.405)} | 0.037 |
| ^{‡}CI, Vertrauensbereich. | | | | |
| ^{†}Kategorievariable. | | | | |
| ^{††}Kategorievariable (Schnittpunkt 33.33 Perzentile). | | | | |
| ^{±}methyliert und geringe Expression vs methyliert oder geringe Expression vs unmethylierte und hohe Expression. | | | | |

## Patentansprüche

1. Verfahren zur Diagnose von oder zur Prognosestellung bei oder zur gleichzeitigen Diagnose von und Prognosestellung bei Eierstockkrebs, **dadurch gekennzeichnet, dass** die Promotor-Hypermethylierung des Markers TUSC3 in einer zu untersuchenden biologischen Probe, vorzugsweise in einer zu untersuchenden Gewebeprobe oder biologischen Flüssigkeit gemessen wird, und bei Feststellung der Hypermethylierung eine Erkrankung bzw. eine progressive Erkrankung diagnostiziert wird.

2. Verfahren nach Anspruch 1, **dadurch gekennzeichnet, dass** es folgende Schritte umfasst:
- Isolation der DNA aus der biologischen Probe, vorzugsweise der Gewebeprobe oder der biologischen Flüssigkeit
- Durchführung eines PCR-basierenden Methylierungsnachweises
- Analyse der gewonnenen PCR-Produkte.

3. Verfahren nach Anspruch 2, **dadurch gekennzeichnet, dass** der PCR-basierende Methylierungsnachweis mit methylierungsspezifischer PCR erfolgt.

4. Verfahren nach Anspruch 3, **dadurch gekennzeichnet, dass** bei der methylierungsspezifischen PCR für den Nachweis methylierter DNA eine PCR mit einem der folgenden Primerpaare durchgeführt wird:
- 5'-TTTTCGGTGAATCGGATG-3' und
5'-TACGCGCCCAACTCCTA-3',
- 5'-GGTTTAGTTAGCGGGTTTTCGG-3' und
5'-AAACAAATACCGCAACCGCCG-3' oder
- 5'-GGTCGGGTAGGCGTGGTGCG-3' und
5'-CCGCCCCGCTTACCTACGACGT-3'.

5. Verfahren nach Anspruch 4, **dadurch gekennzeichnet, dass** zusätzlich für den Nachweis nicht methylierter DNA eine PCR mit einem der folgenden Primerpaare durchgeführt wird:
- 5'-GGGTTTAGTTAGTGGGTTTTTGGAG-3' und
5'-AAACAAATACCACAACCACCACCC-3' oder
- 5'-GAGGTTGGTTGGGTAGGTGTGGTGTG-3' und
5'-CACAACCACCACCCCACTTACCTACAACAT-3'.

6. Verwendung von PCR-Primer, die für die Durchführung einer methylierungsspezifischen PCR in der TUSC3-Promotorregion geeignet sind, in einem Verfahren zur Diagnose von oder Prognosestellung bei oder zur gleichzeitigen Diagnose von und Prognosestellung bei Eierstockkrebs nach Anspruch 1.

7. Verwendung nach Anspruch 6, **dadurch gekennzeichnet, dass** Verfahren zusätzlich Mittel zum Nachweis entstandener PCR-Produkte, wie eine Hybridisierungssonde, enthält.

## Claims

1. A method for diagnosis or prognosis or simultaneous diagnosis and prognosis of ovarian cancer, **characterized in that** the promoter hypermethylation of the marker TUSC3 is measured in a biological sample to be examined, preferably in a tissue sample or biological fluid to be examined, and upon determination of said hypermethylation a disease or a progressive disease, respectively, is diagnosed.

2. The method according to claim 1, **characterized in that** it comprises the following steps:
- isolation of the DNA from said biological sample, preferably said tissue sample or said biological fluid,
- performance of a PCR-based methylation detection,
- analysis of the gained PCR products.

3. The method according to claim 2, **characterized in that** said PCR-based methylation detection is undertaken using methylation-specific PCR.

4. The method according to claim 3, **characterized in that** upon said methylation-specific PCR for detection of methylated DNA, PCR is performed with one of the following primer pairs:
- 5'-TTTTCGGTGAATCGGATG-3' and
5'-TACGCGCCCAACTCCTA-3',
- 5'-GGTTTAGTTAGCGGGTTTTCGG-3' and
5'-AAACAAATACCGCAACCGCCG-3', or
- 5'-GGTCGGGTAGGCGTGGTGCG-3' and
5'-CCGCCCCGCTTACCTACGACGT-3'.

5. The method according to claim 4, **characterized in that** additionally, for detection of non-methylated DNA, PCR is performed with one of the following primer pairs:
- 5'-GGGTTTAGTTAGTGGGTTTTTGGAG-3' and
5'-AAACAAATACCACAACCACCACCC-3', or
- 5'-GAGGTTGGTTGGGTAGGTGTGGTGTG-3' and
5'-CACAACCACCACCCCACTTACCTACAACAT-3'.

6. Use of PCR primers suitable for performance of a methylation-specific PCR in the TUSC3 promoter region in a method for diagnosis or prognosis or simultaneous diagnosis and prognosis of ovarian cancer according to claim 1.

7. Use according to claim 6, **characterized in that** said method additionally includes means for detection of resulting PCR products, like a hybridization probe.

## Revendications

1. Procédé pour le diagnostic de ou pour l'établissement de pronostic en cas de ou pour le diagnostic de et l'établissement de pronostic parallèles en cas de cancer des ovaires, **caractérisé en ce que** l'hyperméthylation du promoteur du marqueur TUSC3 est mesurée dans un échantillon biologique à analyser, de préférence dans un échantillon tissulaire ou un fluide biologique à analyser, et en cas de constatation de l'hyperméthylation, une maladie, respectivement, une maladie progressive est diagnostiquée.

2. Procédé selon la revendication 1, **caractérisé en ce qu'**il comprend les étapes suivantes :
- isoler l'ADN de l'échantillon biologique, de préférence l'échantillon tissulaire ou le fluide biologique
- procéder à une preuve de méthylation par PCR
- analyser les produits PCR obtenus.

3. Procédé selon la revendication 2, **caractérisé en ce que** la preuve de méthylation par PCR s'effectue avec une PCR spécifique à la méthylation.

4. Procédé selon la revendication 3, **caractérisé en ce qu'**en ce qui concerne la PCR spécifique à la méthylation pour apporter la preuve d'ADN méthylée, on effectue une PCR avec l'une des paires d'amorces suivantes :
- 5'-TTTTCGGTGAATCGGATG-3' et
5'-TACGCGCCCAACTCCTA-3',
- 5'-GGTTTAGTTAGCGGGTTTTCGG-3' et
5'-AAACAAATACCGCAACCGCCG-3' ou
- 5'-GGTCGGGTAGGCGTGGTGCG-3' et
5'-CCGCCCCGCTTACCTACGACGT-3'.

5. Procédé selon la revendication 4, **caractérisé en ce que** pour apporter la preuve d'ADN non méthylée, on effectue en plus une PCR avec l'une des paires d'amorces suivantes :
- 5'-GGGTTTAGTTAGTGGGTTTTTGGAG-3' et
5'-AAACAAATACCACAACCACCACCC-3' ou
- 5'-GAGGTTGGTTGGGTAGGTGTGGTGTG-3' et
5'-CACAACCACCACCCCACTTACCTACAACAT-3'.

6. Emploi d'amorces de PCR qui sont appropriées pour effectuer une PCR spécifique à la méthylation dans la région promotrice TUSC3, dans un procédé pour le diagnostic de ou pour l'établissement de pronostic en cas de ou pour le diagnostic de et l'établissement de pronostic parallèles en cas de cancer des ovaires selon la revendication 1.

7. Emploi selon la revendication 6, **caractérisé en ce que** le procédé comporte en outre des moyens pour apporter la preuve des produits de PCR obtenus, comme une sonde d'hybridation.
